# EUROPÄISCHE PATENTSCHRIFT

(11) **EP 3 222 031 B1**
(45) Veröffentlichungstag und Bekanntmachung des Hinweises auf die Patenterteilung: **27.10.2021**
(21) Anmeldenummer: 15813699.4
(22) Anmeldetag: 23.11.2015
(51) Int. Cl.: H04N 5/232, H04N 5/32, A61B 6/14

(54) **VERFAHREN UND VORRICHTUNG ZUR BEREITSTELLUNG VON ZWEI DIGITALEN PANORAMASCHICHTBILDERN**
METHOD AND APPARATUS FOR PROVIDING TWO DIGITAL PANORAMIC LAYER IMAGES
PROCÉDÉ ET DISPOSITIF DE PRODUCTION DE DEUX IMAGES NUMÉRIQUES À COUCHE PANORAMIQUE

(30) Priorität: 21.11.2014 DE 102014223802
(43) Veröffentlichungstag der Anmeldung: 27.09.2017
(73) Patentinhaber: Sirona Dental Systems GmbH, 64625 Bensheim (DE)
(72) Erfinder: LINDENBERG, Kai, 64395 Wersau (DE)
(74) Vertreter: Özer, Alpdeniz
(86) Internationale Anmeldenummer: PCT/EP2015/077308
(87) Internationale Veröffentlichungsnummer: WO 2016/079337

(56) Entgegenhaltungen:
- EP-A1- 2 762 078
- US-A1- 2009 274 997
- US-A1- 2012 328 071
- DESERNO T M ET AL: "A posteriori registration and subtraction of panoramic compared with intraoral radiography", ORAL SURGERY, ORAL MEDICINE, ORAL PATHOLOGY, ORAL RADIOLOGY AND ENDODONTICS, MOSBY-YEAR BOOK, ST. LOUIS, MO, US, Bd. 108, Nr. 2, 1. August 2009 (2009-08-01), Seiten e39-e45, XP026319171, ISSN: 1079-2104, DOI: 10.1016/J.TRIPLEO.2009.03.036 [gefunden am 2009-07-15]
- MEKKY N E ET AL: "A new dental panoramic X-ray image registration technique using hybrid and hierarchical strategies", COMPUTER ENGINEERING AND SYSTEMS (ICCES), 2010 INTERNATIONAL CONFERENCE ON, IEEE, PISCATAWAY, NJ, USA, 30. November 2010 (2010-11-30), Seiten 361-367, XP031840024, ISBN: 978-1-4244-7040-2

## Beschreibung

### Technisches Gebiet

Die Erfindung betrifft ein Verfahren zur Bereitstellung eines ersten und eines zweiten digitalen Panoramaschichtbilds, wobei die beiden Panoramaschichtbilder jeweils aus während eines zumindest teilweisen Umlaufs einer Aufnahmeeinheit um ein aufzunehmendes Objekt detektierten Projektionen gemäß einer Berechnungsvorschrift und unter Ausbildung einer Panoramaschicht erzeugt werden.

### Stand der Technik

In der dentalen Röntgendiagnostik ist es häufig notwendig, Verlaufskontrollen durchzuführen, beispielsweise vorher-/nachher-Vergleiche, Therapieverläufe oder Wachstumsverläufe. Werden solchen Vergleichen digitale Panoramaschichtaufnahmen zugrunde gelegt, so gestaltet sich das Vergleichen häufig schwierig, da die geometrische Verteilung von Gewebestrukturen in den Bilder sehr kritisch von der Positionierung des Patienten abhängt und diese häufig nicht in der notwendigen Genauigkeit rekonstruiert werden kann.

Die Aufgabe der vorliegenden Erfindung ist es daher, ein Verfahren bereitzustellen, welches für einen Vergleich optimierte digitale Panoramaschichtbilder bereitstellt. Reference is made to Deserno T. M. et al, "A posteriori registration and subtraction of panoramic compared with intraoral radiography", Oral Surgery, Oral Medicine, Oral Pathology, Oral Radiology and Endodontics, Mosby-year Yook, St. Louis, MO, US, vol. 108, no. 2, 1. August 2009, pages e39 - e45, ISSN 1079-2104.

EP2762078 A1 discloses an image processor and image processing method.

US2012/0328071 A1 discloses a radiation imaging apparatus amd imaging method using radiation.

### Offenbarung der Erfindung

Diese Aufgabe wird durch ein Verfahren zur Bereitstellung eines ersten und eines zweiten digitalen Panoramaschichtbilds gemäss Anspruch 1 gelöst.

Digitale Panoramaschichtbilder werden beispielsweise durch Verwendung von CCD- oder CMOS Sensoren erzeugt, die beispielsweise mittels einer Szintillatorschicht in Licht umgewandelte Röntgenstrahlen detektieren. Typischerweise, beispielsweise beim Full Frame Panorama Imaging (FFpan), werden während eines zumindest teilweisen Umlaufs viele Projektionen, beispielsweise mehrere tausend Einzelprojektionen, aus verschiedenen Richtungen erzeugt und jeweils einzeln abgespeichert. Die Überlagerung von verschiedenen Aufnahmerichtungen, die zur Ausbildung der für Panoramaschichtbilder typischen scharfen Schicht, hier Panoramaschicht genannt, bzw. der typischen Verwischungsanteile führt, findet erst nachträglich bei der Erzeugung des Panoramaschichtbilds durch eine entsprechende Addition einzelner Projektionen gemäß einer Berechnungsvorschrift statt.

Dadurch kann das Ergebnis, also das Panoramaschichtbild, bzw. die Panoramaschicht sowie die Verwischungsanteile durch Variieren von Parametern der Berechungsvorschrift verändert werden. Beispielsweise ist der Verlauf bzw. die Lage und die Form der Panoramaschicht in dem zu erzeugenden Panoramaschichtbild durch eine Änderung der Addition der einzelnen Projektionsaufnahmen variabel. Der Verlauf der scharfen Schicht kann beispielsweise durch eine entsprechende Änderung der Berechnungsvorschrift für die Erzeugung um einige Millimeter in translatorischer Richtung oder im Bereich von einigen Grad rotatorisch verschoben bzw. gedreht werden. Auch die Form, z.B. die Krümmung und/oder die Dicke der Schicht kann durch eine entsprechende Änderung der Berechnungsvorschrift für die Erzeugung verändert werden. Die Form der Panoramaschicht kann beispielsweise durch eine Spreizung, eine Stauchung oder eine Skalierung im Rahmen der Berechnungsvorschrift verändert werden.

Dieser Spielraum hinsichtlich der Lage bzw. dem Verlauf und der Form der Panoramaschicht in digital zu erzeugenden Panoramaschichtbildern wird genutzt, um eventuelle Unterschiede bei der Positionierung eines Patienten für ein erstes und ein zweites digitales Panoramaschichtbild nachträglich auszugleichen oder zumindest zu verringern. Hierfür werden Unterschiede zwischen den beiden zu vergleichenden Panoramaschichtbildern ermittelt und minimiert, indem die Lage bzw. der Verlauf und/oder die Form der Panoramaschicht in einer der beiden Aufnahmen oder auch in beiden Aufnahmen variiert wird, was durch eine Änderung der Berechnungsvorschrift für das erste und/oder das zweite Panoramaschichtbild erreicht wird.

Wird nur mindestens ein Parameter der Berechnungsvorschrift für das erste oder das zweite Panoramaschichtbild variiert, also nur eines der zwei Panoramaschichtbilder neu berechnet, so ist der Rechenaufwand gering. Dafür ermöglicht eine Minimierung des Unterschieds durch das Variieren der Panoramaschicht für beide Panoramaschichtbilder gegebenenfalls ein besseres Ergebnis, also einen geringeren Unterschied der schlussendlich erhaltenen Panoramaschichtbilder.

Vorteilhafterweise ist der zu ändernde Parameter eine Position und/oder eine Form der Panoramaschicht.

Eine Änderung der Position und/oder der Form, z.B. der Krümmung oder der Dicke der Panoramaschicht, ermöglichen typischerweise eine zuverlässige Minimierung der festgestellten Unterschiede. Der Minimierungsprozess kann beispielsweise nach einer Initialisierung der Schichtform und Schichtlage mit einer Positionssuche starten und anschließend eine Korrektur bzw. Veränderung der Form durchführen. Die beiden Schritte können beispielsweise bis zum Erreichen eines Abbruchkriteriums wiederholt werden. Vorteilhafterweise wird die Position mindestens einer Panoramaschicht hinsichtlich dreier translatorischer und dreier rotatorischer Achsen verändert.

Das Variieren hinsichtlich aller möglichen Freiheitsgrade ermöglicht es, das beste Ergebnis hinsichtlich der Minimierung von Unterschieden zu erhalten. Vorteilhafterweise wird die Form der Panoramaschicht durch eine Spreizung und/oder eine Stauchung und/oder eine Skalierung verändert.

Eine solche Änderung der Form der Panoramaschichtaufnahme kann beispielsweise durch eine entsprechende Änderung der Additionsvorschriften für die Erzeugung der Panoramaschichtaufnahme erreicht werden. Eine solche Änderung ermöglicht eine Minimierung von Unterschieden zwischen den beiden Panoramaschichtaufnahmen insbesondere dann, wenn der zu vermessende Kiefer zwischen den Zeitpunkten, zu denen die zu vergleichenden Aufnahmen erzeugt wurde, seine Größe verändert hat, also gewachsen ist.

Vorteilhafterweise werden die Unterschiede mittels eines Optimierungsverfahrens minimiert.

Der Einsatz von Optimierungsverfahren ermöglicht ein zuverlässiges Auffinden der optimalen oder zumindest sehr geeigneten Lage und/oder Form der Panoramaschicht in dem ersten und/oder zweiten Panoramaschichtbild. Vorteilhafterweise ist das Optimierungsverfahren ein Brute-Force-Suchverfahren oder ein Gradientenverfahren oder ein Struktursuchverfahren.

Dies sind Beispiele für besonders geeignete Optimierungsverfahren, mit deren Hilfe die optimale Lage und/oder Form der Panoramaschicht in dem ersten und/oder zweiten Panoramaschichtbild einfach bestimmt werden kann.

Vorteilhafterweise wird die Panoramaschicht in mindestens zwei unterschiedlichen Bereichen des Panoramaschichtbilds unterschiedlich variiert.

Hierdurch kann beispielsweise die Lage und/oder die Form der Panoramaschicht an die Gegebenheiten des Objekts angepasst werden. Bei einer Vermessung des Kiefers eines Menschen kann hierdurch die Lage der Panoramaschicht beispielsweise im Bereich des Oberkiefers an dessen Verlauf angepasst werden, während sie im Bereich des Unterkiefers an dessen Verlauf angepasst werden kann.

Weiterhin betrifft die Erfindung eine Vorrichtung zur Bereitstellung eines ersten und eines zweiten digitalen Panoramaschichtbilds. Die Vorrichtung umfasst eine Recheneinheit, ein dentale Röntgengerät mit einer Röntgenquelle und mindestens einem digitalen Röntgendetektor, die jeweils zumindest teilweise um einen Aufnahmeraum herum bewegbar sind, und ein Mittel zur Übertragung von mit dem Detektor detektierten Projektionen an die Recheneinheit. Die Recheneinheit ist dazu ausgebildet, das erste und das zweite Panoramaschichtbild jeweils aus mehreren während eines Umlaufs detektierten Projektionen gemäß einer Berechnungsvorschrift und unter Ausbildung einer Panoramaschicht zu erzeugen und das vorab beschriebene Verfahren auszuführen.

### Kurze Beschreibung der Zeichnungen

Das erfindungsgemäße Verfahren sowie die erfindungsgemäße Vorrichtung werden anhand der Zeichnung erläutert. Es zeigen:
- Fig. 1: ein Vorrichtung zur Bereitstellen eines ersten und eines zweiten digitalen Panoramaschichtbilds;
- Fig. 2: ein Verfahren zur Bereitstellung eines ersten und eines zweiten digitaler Panoramaschichtbilds. Ausführungsformen der Erfindung

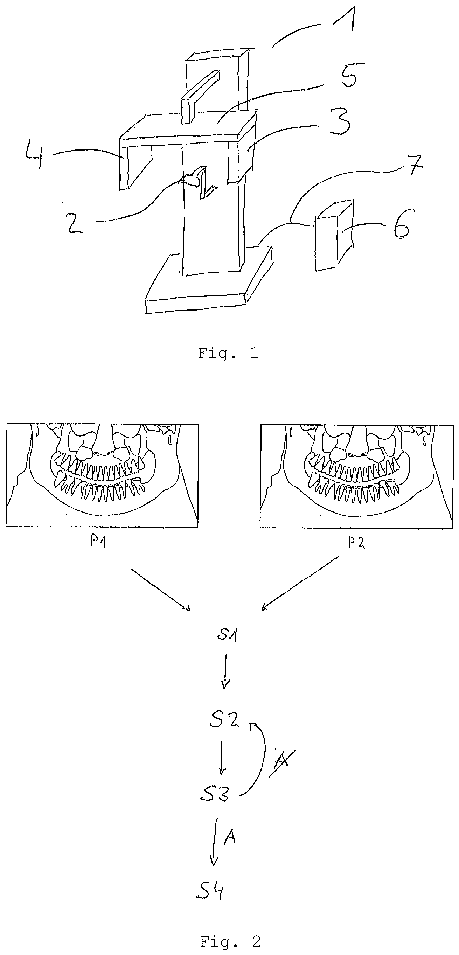

In Fig. 1 ist ein dentales Röntgengerät 1 zur Erzeugung von Panoramaschichtbildern P1, P2 skizziert. Ein Patient wird typischerweise mittels einer Positioniervorrichtung 2 in einem Aufnahmeraum zwischen einer Röntgenquelle 3 und mindestens einem digitalen Röntgendetektor 4 positioniert, wobei die Röntgenquelle 3 und der Röntgendetektor 4 an einem drehbaren Arm 5 angeordnet sind und zur Erzeugung eines Panoramaschichtbilds P1, P2 zumindest teilweise beispielsweise kreisförmig um einen Aufnahmeraum und einen darin positionierten Patienten herum bewegbar sind. Während dieses zumindest teilweisen Umlaufs wird für verschiedene Winkelstellungen des Arms 5 relativ zum positionierten Patienten mittels des Röntgendetektors 4 jeweils eine Projektion detektiert und mittels eines Übertragungsmittels 7 an eine Recheneinheit 6 übermittelt. Während eines vollen Umlaufs können beispielsweise 3000 solcher Projektionen detektiert werden.

Mittels der Recheneinheit 6 wird im Anschluss an den Umlauf ein Panoramaschichtbild P1 , P2 aus den detektierten Projektionen erzeugt, indem diese gemäß einer Berechnungsvorschrift so addiert bzw. überlagert werden, dass sich ein klassisches Panoramaschichtbild mit einer scharfen Schicht, der Panoramaschicht, und einem Verwischungsanteilen ergibt. Je mehr Projektionen erzeugt bzw. dem Panoramaschichtbild zugrunde gelegt werden, um so ausgeprägter ist die sich ergebende Panoramaschicht.

Um einen Wachstums- oder Therapieverlauf zu überwachen kann beispielsweise in zeitlichen Abständen jeweils ein solches digitales Panoramaschichtbild desselben Patienten erzeugt werden. Es wird also zu einem ersten Zeitpunkt ein erstes Panoramaschichtbild P1 aus während eines ersten Umlaufs erzeugten Projektionen und nach einiger Zeit ein zweites Panoramaschichtbild P2 aus während eines zweiten Umlaufs erzeugten Projektionen desselben Patienten erzeugt, wobei der Patienten für beide Panoramaschichtbilder P1, P2 möglichst gleich Positioniert werden sollte und jeweils die selbe Berechnungsvorschrift zur Erzeugung der beiden Panoramaschichtbilder P1, P2 aus den jeweiligen Projektionen verwendet wird.

Um die beiden Panoramaschichtbilder für den Vergleich weiter zu optimieren wird erfindungsgemäß der Unterschied zwischen den Panoramaschichtbildern P1, P2 minimiert. Hierdurch sollen Unterschiede, welche lediglich auf einer unterschiedlichen Positionierung des Patienten oder unterschiedlicher Bedingungen bei der Aufnahme beruhen möglichst vollständig oder zumindest so weit wie möglich eliminiert werden, so dass reale Unterschiede aufzunehmenden Objekts, z.B. des Patienten, zu den beiden Aufnahmezeitpunkten in den Aufnahmen einfacher und deutlicher zu erkennen sind.

Hierfür werden beispielsweise ausgehend von zwei nach der gleichen Berechnungsvorschrift und unter Ausbildung von jeweils einer Panoramaschicht aus den jeweiligen Projektionsaufnahmen erzeugten Panoramaschichtbildern P1, P2 in einem ersten Schritt S1 Unterschiede U1 zwischen den beiden Panoramaschichtbildern P1, P2 ermittelt. In einem zweiten Schritt S2 wird beispielsweise das erste Panoramaschichtbild P1 erneut aus den während des ersten Umlaufs detektierten Projektionsaufnahmen erzeugt, wobei eine Lage und/oder eine Form der Panoramaschicht durch Änderung der Berechnungsvorschrift variiert wird. Schritt S2 kann auch vorsehen nur das zweite Panoramaschichtbild P2 erneut mit geänderter Berechnungsvorschrift zu berechnen oder beide Panoramaschichtbilder P1, P2 mit jeweils neuer Berechnungsvorschrift neu zu erzeugen.

Im anschließenden Schritt S3 wird je nach Wahl des Schritts S2 das neu erzeugte erste Panoramaschichtbild P1 mit dem zweiten Panoramaschichtbild P2 bzw. das erste Panoramaschichtbild P1 mit dem neu erzeugten zweiten Panoramaschichtbild P2 bzw. das neu erzeugte erste Panoramaschichtbild P1 mit dem neu erzeugten zweiten Panoramaschichtbild P2 verglichen und Unterschiede U2 ermittelt.

Schritte S2 und S3 werden so lange wiederholt, bis der ermittelte Unterschied zwischen den aktuellen Panoramaschichtbildern P1, P2 möglichst gering bzw. nicht mehr vorhanden ist bzw. eine Abbruchbedingung A erfüllt ist.

Es kann beispielsweise ein Grenzwert für die Unterschiede U1, U2 vorgesehen werden und ein Abbruch der Minimierung erfolgen, sobald dieser Grenzwert unterschritten ist. Oder es wird ein Optimierungsverfahren zur Minimierung der Unterschiede U1, U2 eingesetzt, welches eine andere Abbruchbedingung hat.

Im abschließenden Schritt S4 werden die beiden aktuellen Panoramaschichtbilder P1, P2 abgelegt und/oder ausgegeben, um sie einem Anwender zum Vergleich zur Verfügung zu stellen.

Der Unterschied zwischen den Panoramaschichtbildern P1, P2 kann beispielsweise mittels eines Mutual-Information-Verfahrens ermittelt bzw. bewertetet werden. Eine Minimierung kann dann beispielsweise folgende Verfahrensschirtte umfassen: Ein erster Paramter, z.B. die Position, wird variiert, z.B. durch Translation. Mindestens eines der beiden Panoramaschichtbilder P1, P2 wird mit dem geänderten Parameter neu berechent. Der Unterschied zwischen den neu berechneten Panoramaschichtbildern P1 und P2 oder zwischen dem neu berechneten und dem unveränderten Panoramaschichtbild P1, P2 wird gemäß dem Mutual-Information-Verfahren bewertet. Diese Schritte werden bis zum Erreichen eines Minimus wiederholt. Anschließend wird eine entsprechende Minimierung des Unterschieds beispielsweise hinsichtlich eines oder mehrerer weiterer Parameters vorgenommen. Wiederum anschließend wird zur Minimierung des Unterschieds z.B. wieder der erste Parameter, dann der zweite Parameter usw. variiert, bis ein allgemeines Minimum des Unterschieds bzw. ein allgemeines Maximum der Gleichheit gefunden wurde bzw. erreicht ist.

### Bezugszeichen

- 1: digitales Röntgengerät
- 2: Positioniervorrichtung
- 3: Röntgenquelle
- 4: Röntgendetektor
- 5: drehbarer Arm
- 6: Recheneinheit
- 7: Übertragungsmittel
- A: Abbruchbedingung
- P1: Panoramaschichtbild
- P2: Panoramaschichtbild
- S1: Schritt 1
- S2: Schritt 2
- S3: Schritt 3
- S4: Schritt 4
- U1: Unterschiede
- U2: Unterschiede

## Patentansprüche

1. Verfahren zur Bereitstellung eines ersten und eines zweiten digitalen Panoramaschichtbilds (P1, P2), wobei für das erste und das zweite Panoramaschichtbild (P1, P2) jeweils während eines zumindest teilweisen Umlaufs einer Aufnahmeeinheit (1) um ein aufzunehmendes Objekt mehrere Projektionen mittels mindestens eines digitalen Detektors (4) detektiert und gespeichert werden und das erste und das zweite Panoramaschichtbild (P1, P2) jeweils aus den mehreren Projektionen gemäß einer Berechnungsvorschrift und unter Ausbildung einer Panoramaschicht erzeugt werden, **dadurch gekennzeichnet, dass** Unterschiede zwischen dem ersten und dem zweiten Panoramaschichtbild (P1, P2) durch Veränderung mindestens einer Position und/oder einer Form der Panoramaschicht bei einer erneuten Erzeugung des ersten und/oder des zweiten Panoramaschichtbilds (P1, P2) mittels eines Optimierungsverfahrens minimiert werden.

2. Verfahren nach Anspruch 1, **dadurch gekennzeichnet, dass** die Position der Panoramaschicht hinsichtlich dreier translatorischer und dreier rotatorischer Achsen verändert wird.

3. Verfahren nach Anspruch 2, **dadurch gekennzeichnet, dass** die Form der Panoramaschicht durch eine Spreizung und/oder eine Stauchung und/oder eine Skalierung verändert wird.

4. Verfahren nach einem der Ansprüche 1 bis 3, **dadurch gekennzeichnet, dass** das Optimierungsverfahren ein Brute-Force-Suchverfahren oder ein Gradientenverfahren oder ein Struktursuchverfahren ist.

5. Verfahren nach einem der Ansprüche 1 bis 4, **dadurch gekennzeichnet, dass** die Panoramaschicht in mindestens zwei unterschiedlichen Bereichen des Panoramaschichtbilds unterschiedlich variiert wird.

6. Vorrichtung zur Bereitstellung eines ersten und eines zweiten digitalen Panoramaschichtbilds (P1, P2), umfassend eine Recheneinheit (6), ein dentales Röntgengerät (1) mit einer Röntgenquelle (3) und mindestens einem digitalen Röntgendetektor (4), die jeweils zumindest teilweise um einen Aufnahmeraum herum bewegbarer sind, und Mittel (7) zur Übertragung von mit dem Detektor (4) detektierten Projektionen an die Recheneinheit (6), wobei die Recheneinheit (6) dazu ausgebildet ist, das erste und das zweite Panoramaschichtbild (P1, P2) jeweils aus mehreren während eines Umlaufs detektierten Projektionen gemäß einer Berechnungsvorschrift und unter Ausbildung einer Panoramaschicht zu erzeugen, **dadurch gekennzeichnet, dass** die Recheneinheit (6) dazu ausgebildet ist, das Verfahren nach einem der Ansprüche 1 bis 5 auszuführen.

## Claims

1. Method for providing a first and a second digital panoramic tomogram (P1, P2),
wherein multiple projections are detected and stored for the first and second panoramic tomograms (P1, P2) by means of at least one digital detector (4) in each case during an at least partial revolution of a recording unit (1) around an object to be recorded and the first and second panoramic tomograms (P1, P2) are in each case generated from the multiple projections according to a calculation rule and with the formation of a panoramic slice, **characterised in that** differences between the first and second panoramic tomograms (P1, P2) are minimised by modifying at least one position and/or shape of the panoramic slice when newly generating the first and/or second panoramic tomogram (P1, P2) by means of an optimisation method.

2. Method according to claim 1, **characterised in that** the position of the panoramic slice is modified in respect of three translational and three rotational axes.

3. Method according to claim 2, **characterised in that** the shape of the panoramic slice is modified by spreading and/or compressing and/or scaling.

4. Method according to any one of claims 1 to 3, **characterised in that** the optimisation method is a brute-force search method or a gradient method or a structure search method.

5. Method according to any one of claims 1 to 4, **characterised in that** the panoramic slice is varied differently in at least two different regions of the panoramic tomogram.

6. Apparatus for providing a first and a second digital panoramic tomogram (P1, P2), comprising a computing unit (6), a dental X-ray instrument (1) having an X-ray source (3) and at least one digital X-ray detector (4), each of which is at least partially movable around a recording space, and means (7) for transmitting projections detected by the detector (4) to the computing unit (6), wherein the computing unit (6) is designed to generate the first and second panoramic tomograms (P1, P2) in each case from multiple projections detected during a revolution according to a calculation rule and with the formation of a panoramic slice, **characterised in that** the computing unit (6) is designed to carry out the method according to any one of claims 1 to 5.

## Revendications

1. Procédé de fourniture d'une première et d'une seconde image de couche panoramique (P1, P2) numérique, dans lequel pour chacune de la première et la seconde image de couche panoramique (P1, P2), lors d'une rotation au moins partielle d'une unité d'enregistrement (1) autour d'un objet à enregistrer, plusieurs projections au moyen d'au moins un détecteur (4) numérique sont détectées et mémorisées et chacune de la première et la seconde image de couche panoramique (P1, P2) est générée à partir de plusieurs projections selon une règle de calcul et pour former une couche panoramique, **caractérisé en ce que** les différences entre la première et la seconde image de couche panoramique (P1, P2) peuvent être diminuées par changement d'au moins une position et/ou d'une forme de la couche panoramique lors d'une nouvelle génération de la première et/ou de la seconde image (P1, P2) de couche panoramique au moyen d'un procédé d'optimisation.

2. Procédé selon la revendication 1, **caractérisé en ce que** la position de la couche panoramique est modifiée par rapport à trois axes de translation et trois axes de rotation.

3. Procédé selon la revendication 2, **caractérisé en ce que** la forme de la couche panoramique est modifiée par étalement et/ou par compression et/ou par mise à l'échelle.

4. Procédé selon l'une quelconque des revendications 1 à 3, **caractérisé en ce que** le procédé d'optimisation est un procédé de recherche par force brute ou un procédé du gradient ou un procédé de recherche de structure.

5. Procédé selon l'une quelconque des revendications 1 à 4, **caractérisé en ce que** la couche panoramique est modifiée différemment dans au moins deux zones différentes de l'image de couche panoramique.

6. Dispositif de fourniture d'une première et d'une seconde image de couche panoramique (P1, P2) numérique, comprenant une unité de calcul (6), un appareil dentaire à rayons X (1) doté d'une source de rayons X (3) et au moins un détecteur de rayons X (4) numérique, dont chacun peut au moins partiellement déplacé autour d'un espace d'enregistrement, et des moyens (7) pour transmettre les projections détectées par le détecteur (4) à l'unité de calcul (6), dans lequel l'unité de calcul (6) est conçue pour générer chacune de la première et la seconde image de coupe panoramique (P1, P2), à partir de plusieurs projections détectées lors d'une rotation selon une règle de calcul et avec formation d'une couche panoramique, **caractérisé en ce que** l'unité de calcul (6) est conçue pour mettre en œuvre le procédé selon l'une quelconque des revendications 1 à 5.
